(19) Europäisches Patentamt
European Patent Office
Office européen des brevets

(11) Veröffentlichungsnummer : **0 075 840**
**B1**

(12) **EUROPÄISCHE PATENTSCHRIFT**

(45) Veröffentlichungstag der Patentschrift : **11.11.87**

(21) Anmeldenummer : **82108719.4**

(22) Anmeldetag : **21.09.82**

(51) Int. Cl.⁴ : **C 07 D513/04**, C 07 D471/04, C 07 D498/04, A 01 N 43/90 // (C07D513/04, 277:00, 221:00),(C07D471/04, 235:00, 221:00),(C07D498/04, 263:00, 221:00)

(54) **Heterocyclische Phenyläther, Verfahren zu deren Herstellung und diese enthaltende herbizide Mittel.**

(30) Priorität : **24.09.81 DE 3137996**

(43) Veröffentlichungstag der Anmeldung : **06.04.83 Patentblatt 83/14**

(45) Bekanntmachung des Hinweises auf die Patenterteilung : **11.11.87 Patentblatt 87/46**

(84) Benannte Vertragsstaaten : **AT BE CH DE FR GB IT LI NL**

(56) Entgegenhaltungen :
**DE-A- 1 670 750**
**DE-A- 2 640 730**
**Die Akte enthält technische Angaben, die nach dem Eingang der Anmeldung eingereicht wurden und die nicht in dieser Patentschrift enthalten sind.**

(73) Patentinhaber : **HOECHST AKTIENGESELLSCHAFT**
**Postfach 80 03 20**
**D-6230 Frankfurt am Main 80 (DE)**

(72) Erfinder : **Willms, Lothar, Dr.**
**Grüner Weg 4**
**D-5463 Unkel (DE)**
Erfinder : **Handte, Reinhard, Dr.**
**Breckenheimer Strasse 45**
**D-6238 Hofheim am Taunus (DE)**
Erfinder : **Mildenberger, Hilmar, Dr.**
**Fasanenstrasse 24**
**D-6233 Kelkheim (Taunus) (DE)**
Erfinder : **Bauer, Klaus, Dr.**
**Kolpingstrasse 7**
**D-6054 Rodgau (DE)**
Erfinder : **Bieringer, Hermann, Dr.**
**Eichenweg 26**
**D-6239 Eppstein/Taunus (DE)**
Erfinder : **Bürstell, Helmut, Dr.**
**Am Hohlacker 65**
**D-6000 Frankfurt am Main 50 (DE)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Jouve, 18, rue St-Denis, 75001 Paris, France

**Beschreibung**

Aus DE-A-2 640 730 und DE-A-1 670 750 sind herbizid wirksame heterocyclische Phenyläther und Imidazopyridine bekannt. Gegenstand der vorliegenden Erfindung sind neue heterocyclisch substituierte 4-Phenoxyalkancarbonsäurederivate der allgemeinen Formel

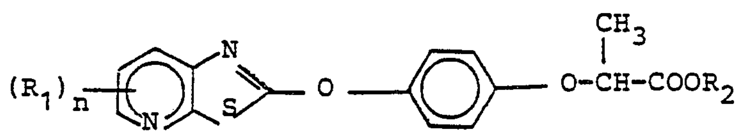

(I)

worin
$R_1$ Halogen,
n 0 bis 3,
$R_2$ H, $(C_1$-$C_{12})$-Alkyl,
oder ein Kationäquivalent einer organischen oder anorganischen Base bedeuten.

Alkylreste können sowohl geradkettig als auch verzweigt sein. Besonders bevorzugt sind Verbindungen mit $R_1$ = Halogen und Z = COOH oder $COO(C_1$-$C_4)$ Alkyl.

Gegenstand der Erfindung sind ferner Verfahren zur Herstellung der Verbindungen der Formel I, dadurch gekennzeichnet, daß man

a) Verbindungen der Formel

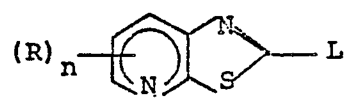

(II)

worin L eine Abgangsgruppe wie z. B. Halogen, $(C_1$-$C_4)$-Alkylsulfonyl, Phenylsulfonyl, $(C_1$-$C_4)$-Alkoxycarbonylmethylsulfonyl, $(C_1$-$C_4)$-Alkylsulfinyl, $(C_1$-$C_4)$-Alkylthio sowie den Mesyl- oder Tosylrest bedeutet, wie Verbindungen der Formel

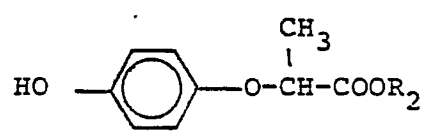

(III)

oder

b) Verbindungen der Formel

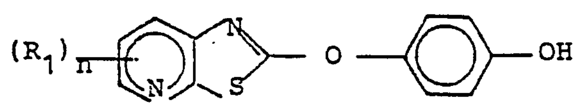

(IV)

mit Verbindungen der Formel

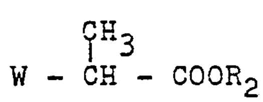

(V)

worin W für Halogen (vorzugsweise Chlor oder Brom) oder den Mesyl- bzw. Tosylrest steht, umsetzt oder

c) die nach Verfahren a) oder b) erhaltenen Verbindungen durch Verseifung, Salzbildung, Veresterung oder Umesterung in andere erfindungsgemäße Verbindungen der Formel I überführt.

Zu a) und b)

Die Umsetzung der Verbindungen II und III sowie IV und V erfolgt vorzugsweise in inerten aprotischen Lösungsmitteln wie aliphatischen oder aromatischen Kohlenwasserstoffen (wie z. B. Benzol, Toluol, Xylol), Säurenitrilen wie z. B. Acetonitril oder Propionitril, Ketonen wie z. B. Aceton, Methyläthylketon oder Methylisobutylketon, Säureamiden wie z. B. Dimethylformamid, Dimethylacetamid, N-Methylpyrrolidon oder Hexamethylphosphorsäuretriamid oder in Dimethylsulfoxid bei Temperaturen zwischen 30° und 250 °C bzw. der Siedetemperatur des Lösungsmittels, vorzugsweise zwischen 60° und 160 °C, in

Gegenwart anorganischer oder organischer Basen wie z. B. Metallalkoholaten, tertiären Aminen, Alkali- oder Erdalkalicarbonaten und Hydroxyden (NaOH, KOH).

Zu c)

Die Umesterung geschieht durch saure oder basische Katalyse. Man setzt den Alkohol, der in den Ester eingeführt werden soll, zweckmäßigerweise im Überschuß zu und destilliert den freiwerdenden, niedriger siedenden Alkohol laufend in dem Maße ab, in dem er bei der Umesterung gebildet wird.

Die für die Herstellung der erfindungsgemäßen Verbindungen der allgemeinen Formel I benötigten Heterocyclen der Formel II werden nach bekannten Verfahren, z. B. aus den entsprechenden 2-Merkaptoverbindungen bzw. 2-Oxoverbindungen durch Halogenierung bzw. aus den entsprechenden 2-Merkaptoverbindungen durch Alkylierung und Oxidation hergestellt (vgl. z. B. Chem. Pharm. Bull 7, 720 (1959) ; Chem. Pharm. Bull 3, 356 (1955) ; J. Pharm. Soc. Japan 71, 920 (1951)).

Die entsprechenden Phenole der Formeln III und IV lassen sich z. B. durch Monoalkylierung von Hydrochinon (J. Org. Chem. 39, S. 214 (1974) bzw. Soc. 1965, 954-73) herstellen.

Für $R_1$ Wasserstoff weisen die Verbindungen der Formel I ein Asymmetriezentrum auf und liegen üblicherweise in racemischer Form vor. Die Racemate lassen sich nach üblichen Methoden in Diastereomere trennen. Ebenso ist es jedoch auch möglich, nach den genannten Verfahren die Verbindungen III und V optisch aktiver Form einzusetzen. Gegenstand der Erfindung sind sowohl die Racemate als auch die optischen Antipoden, besonders deren D-Formen.

Die erfindungsgemäßen Verbindungen der allgemeinen Formel I sind im Vor- und Nachauflaufverfahren gegen ein breites Spektrum von ein- und mehrjährigen Schadgräsern sehr gut wirksam, gleichzeitig werden sie jedoch von zweikeimblättrigen Kulturpflanzen sowie einigen Getreidearten vorzüglich toleriert. Die Verbindungen sind daher zur selektiven Bekämpfung von ein- und mehrjährigen Schadgräsern in Kulturpflanzen geeignet. Solche Schadgräser sind beispielsweise Wildhafer (Avena), Fuchsschwanz (Alopecurus spp.), Rispengras (Poa spp.), Raygras (Lolium spp.), ein- und mehrjährige Wildhirsen (Echinochloa spp.), Setaria spp., Digitaria spp., Panicum spp., Sorghum spp.), Bermudagras (Cynodon spp.) und Quecke (Agropyron spp.).

Bei Anwendung der erfindungsgemäßen Verbindungen in subtoxischen Dosen lassen sich typische wachstumsregulierende Effekte feststellen ; so können beispielsweise der Wuchs der Pflanzen, aber auch die Pflanzeninhaltstoffe beeinflußt werden. Damit eignen sich die Verbindungen als Wachstumsregulatoren in Nutzpflanzenkulturen wie z. B. Getreide, Mais, Zuckerrohr, Tabak, Reis und Sorghum. Andererseits lassen sich auch Pflanzenbestände regulieren wie Kulturrasen oder auch Pflanzengemeinschaften an Wege- und Straßenrändern sowie Zierpflanzen.

Gegenstand der vorliegenden Erfindung sind daher auch herbizide und wuchsregulierende Mittel, die dadurch gekennzeichnet sind, daß sie eine herbizide oder wuchsregulierend wirksame Menge einer Verbindung der allgemeinen Formel I neben üblichen Zusatz- und Formulierungshilfsmitteln enthalten.

Die erfindungsgemäßen Mittel enthalten die Wirkstoffe der Formel I im allgemeinen zu 2-95 Gew.-%. Sie können als benetzbare Pulver, emulgierbare Konzentrate, versprühbare Lösungen, Stäubemittel oder Granulate in den üblichen Zubereitungen angewendet werden.

Benetzbare Pulver sind in Wasser gleichmäßig dispergierbare Präparate, die neben dem Wirkstoff außer einem Verdünnungs- oder Inertstoff noch Netzmittel, z. B. polyoxäthylierte Alkylphenole, polyoxäthylierte Oleyl-, Stearylamine, Alkyl- oder Alkylphenyl-sulfonate und Dispergiermittel, z. B. ligninsulfonsaures Natrium, 2,2'-dinaphthylmethan-6,6'-disulfonsaures Natrium, oder auch oleoylmethyltaurinsaures Natrium enthalten.

Emulgierbare Konzentrate werden durch Auflösen des Wirkstoffes in einem organischen Lösungsmittel, z. B. Butanol, Cyclohexanon, Dimethylformamid, Xylol oder auch höhersiedenden Aromaten und Zusatz eines nichtionischen Netzmittels, beispielsweise eines polyoxäthylierten Alkylphenols oder eine polyoxäthylierten Oleyl- oder Stearylamins, erhalten.

Stäubemittel erhält man durch Vermahlen des Wirkstoffes mit fein verteilten, festen Stoffen, z. B. Talkum, natürlichen Tonen, wie Kaolin, Bentonit, Pyrophillit oder Diatomeenerde.

Versprühbare Lösungen, wie sie vielfach in Sprühdosen gehandelt werden, enthalten den Wirkstoff in einem organischen lösungsmittel gelöst, daneben befindet sich z. B. als Treibmittel ein Gemisch von Fluorchlorkohlenwasserstoffen.

Granulate können entweder durch Verdüsen des Wirkstoffes auf adsorptionsfähiges, granuliertes Inertmaterial hergestellt werden oder durch Aufbringen von Wirkstoffkonzentraten mittels Klebemitteln, z. B. Polyvinylalkohol, polyacrylsaurem Natrium, oder auch Mineralölen auf die Oberfläche von Trägerstoffen, wie Sand, Kaolinite, oder von granuliertem Inertmaterial. Auch können geeignete Wirkstoffe in der für die Herstellung von Düngemittelgranalien üblichen Weise — gewünschtenfalls in Mischung mit Düngemitteln — hergestellt werden.

Bei den herbiziden Mitteln können die Konzentrationen der Wirkstoffe in den handelsüblichen Formulierungen verschieden sein. In benetzbaren Pulvern variiert die Wirkstoffkonzentration z. B. zwischen etwa 10 % und 95 %, der Rest besteht aus den oben angegebenen Formulierungszusätzen. Bei emulgierbaren Konzentraten ist die Wirkstoffkonzentration etwa 10 % bis 80 %. Staubförmige Formulierungen enthalten meistens 5-20 % an Wirkstoff, versprühbare Lösungen etwa 2-20 %. Bei Granulaten

hängt der Wirkstoffgehalt z. T. davon ab, ob die wirksame Verbindung flüssig oder fest vorliegt und welche Granulierhilfsmittel, Füllstoffe usw. verwendet werden.

Zur Anwendung werden die handelsüblichen Konzentrate gegebenenfalls in üblicher Weise verdünnt, z. B. bei benetzbaren Pulvern und emulgierbaren Konzentraten mittels Wasser. Staubförmige und granulierte Zubereitungen sowie versprühbare Lösungen werden vor der Anwendung nicht mehr mit weiteren inerten Stoffen verdünnt. Mit den äußeren Bedingungen wie Temperaturen, Feuchtigkeit u. a. variiert die erforderliche Aufwandmenge. Sie kann innerhalb weiter Grenzen schwanken und liegt für herbizide Mittel bei 0,01 und 10 kg/ha, für wachstumsregulierende Mittel bei 0,001-1 kg/ha.

Die erfindungsgemäßen Wirkstoffe können mit anderen Herbiziden, Insektiziden und Fungiziden kombiniert werden.

## Formulierungsbeispiele

### Beispiel A

Ein emulgierbares Konzentrat wird erhalten aus

15 Gew.-Teilen Wirkstoff
75 Gew.-Teilen Cyclohexanon als Lösungsmittel und
10 Gew.-Teilen oxäthyliertes Nonylphenol (10 AeO) als Emulgator.

### Beispiel B

Ein in Wasser leicht dispergierbares benetzbares Pulver wird erhalten, indem man

25 Gew.-Teile Wirkstoff
64 Gew.-Teile kaolinhaltigen Quarz als Inertstoff
10 Gew.-Teile ligninsulfonsaures Calcium und
1 Gew.-Teil oleoylmethyltaurinsaures Natrium als Netz- und Dispergiermittel
mischt und in einer Stifmühle mahlt.

### Beispiel C

Ein Stäubemittel wird erhalten, indem man

10 Gew.-Teile Wirkstoff, und
90 Gew.-Teile Talkum als Inerstoff

mischt und in einer Schlagmühle zerkleinert.

### Beispiel D

Ein Granulat besteht z. B. aus etwa

2-15 Gew.-Teilen Wirkstoff
98-85 Gew.-Teilen inerten Granulatmaterialien, wie z. B. Attapulgit, Bimsstein, und Quarzsand.

## Herstellungsbeispiele

### Beispiel 1

Ethyl-2[4-(5-chlor-thiazolo [5,4-b] pyridin-2-yloxy)-phenoxy]-propionat

21 g (0,1 Mol) 2-(4'-Hydroxyphenoxy)-propionsäureethylester werden mit 16,6 g (0,12 Mol) Kaliumcarbonat in 250 ml Acetonitril zur Salzbildung 1 Stunde unter Rückfluß erhitzt. Nach Zugabe von 24,9 g (0,1 Mol) 2-Methylsulfonyl-5-chlor-thiazolo [5,4-b] pyridin wird das Reaktionsgemisch weitere 10 Stunden unter Rückfluß erhitzt. Man filtriert heiß vom Salzanteil ab und zieht das Lösungsmittel ab. Der Rückstand wird im Hochvakuum destilliert. Man erhält 33,6 g (89 % d. Th.) an Ethyl-2[4-(5-chlor-thiazolo [5,4-b]-pyridin-2-yloxy)-phenoxy]-propionat vom Siedepunkt 204-207 °C/0,01 mbar, welches nach einiger Zeit durchkristallisiert (Fp. 60-62 °C).

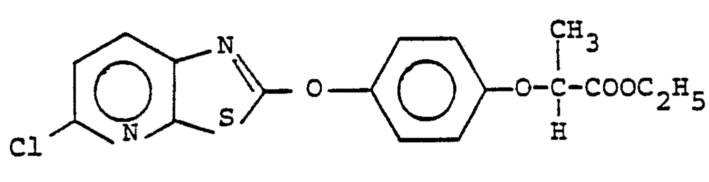

Beispiel 2

Butyl-2-[4-(5-chlor-thiazolo [5,4-b]-pyridin-2-yloxy)-phenoxy]-propionat

a) 2-[4-(5-Chlor-thiazolo [5,4-b]-pyridin-2-yloxy)-phenoxy]-propionsäure

37,8 g (0,1 Mol) Ethyl-2-[4-(5-chlor-thiazolo-[5,4-b]-pyridin-2-yloxy)-phenoxy]-propionat aus Beispiel 1) werden mit 4,4 g (0,11 Mol) Natriumhydroxid in 250 ml Wasser 24 Stunden bei ca. 30 °C gerührt. Die leicht trübe Lösung wird filtriert, anschließend mit 500 ml Toluol versetzt, aud 80 °C erwärmt und mit konz. Salzsäure auf pH 1 gestellt. Die Phasen wurden heiß getrennt, die Wasserphase wird erneut mit 250 ml Toluol heiß ausgerührt, die vereinigten Toluolphasen werden azeotrop getrocknet und auf ca. 250 ml Volumen eingeengt.

b) Herstellung des Säurechlorids von a)

Die nach a) erhaltene Säure in Toluol wird bei ca. 70 °C mit 14,3 mg (0,12 Mol) Thionylchlorid versetzt. Nach der Zugabe wird solange unter Rückfluß erhitzt, bis die Gasentwicklung beendet ist (Dauer ca. 7 h). Man destilliert überschüssiges Thionylchlorid und etwas Toluol ab, die resultierende Lösung des Säurechlorids wird direkt umgesetzt.

c) Umsetzung des Säurechlorids von b)

200 ml Lösung von b) (0,1 Mol) Säurechlorid werden bei ca. 25 °C vorgelegt und mit 11 g (0,11 Mol) Triethylamin und 7,4 g (0,1 Mol) Butanol gelöst in 100 ml Toluol versetzt. Nach der Zugabe wird 2 h bei 60 °C gerührt, abgekühlt und durch Zugabe von 200 ml Wasser das gebildete Hydrochlorid entfernt. Die organische Phase wird nach Trennen getrocknet und eingeengt, der verbleibende Rückstand wird destilliert. Nach Destillation erhält man 34,6 g (85 % der Theorie) an Butyl-2-[4-(5-chlor-thiazolo [5,4-b] pyridin-2-yloxy)-phenoxy]-propionat vom Siedepunkt 225-230°/0,1 mbar.

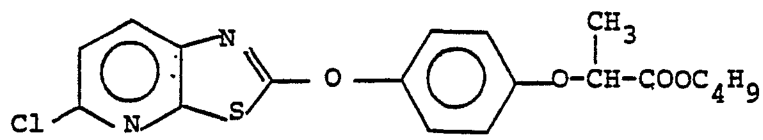

In analoger Weise erhält man gemäß den Herstellungsbeispielen 1) und 2) folgende Verbindungen der allgemeinen Formel

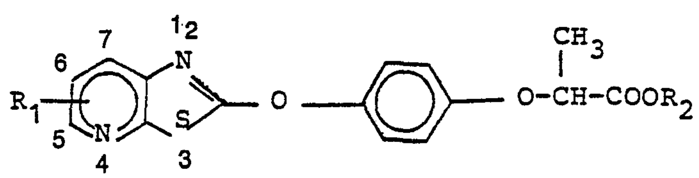

Tabelle 1 : Verbindungen der allg. Formel

| lfd. Beispiel | $R_1$ | $R_2$ | Kp; Fp. |
|---|---|---|---|
| 3 | H | $-C_2H_5$ | Kp 196-198°C/0,1 mbar |
| 4 | 5-Cl | $-CH_3$ | |
| 5 | 5-Cl | $-H$ | |
| 6 | 5-Cl | $-Na$ | |

## Biologische Beispiele

### Beispiel I : Vorauflaufbehandlung

Samen von Gräsern wurden in Töpfen ausgesät und die als Spritzpulver bzw. als Emulsionskonzentrate formulierten erfindungsgemäßen Präparate in verschiedenen Dosierungen auf die Kulturboden-Oberfläche gesprüht. Anschließend wurden die Töpfe für 4 Wochen in einem Gewächshaus aufgestellt und das Resultat der Behandlung (ebenso wie bei den folgenden Beispielen) durch eine Bonitierung nach dem nachstehenden Schema festgehalten :

1 0-20 % Schädigung
2 20-40 % Schädigung
3 40-60 % Schädigung
4 60-80 % Schädigung
5 80-100 % Schädigung

Die erfindungsgemäßen Präparate zeigten eine gute Wirkung gegen einjährige und zum Teil auch gegen mehrjährige Schadgräser, als Testpflanzen wurden verwendet Avena, Alopecurus, Lolium, Echinochloa, Setaria, Agropyron und Cynodon.

Aufwandmengen von 2,4 kg a. i./ha führten führten fast immer zu Schäden im Bereich der Wertzahlen 4 und 5.

Tabelle 1

Vorauflaufbehandlung (Dosierung : 2,4 kg Aktivsubstanz/ha)

| Verbindung (Beispiel) | AVF | ALM | SAL | LOM | ECG | AGR | CND *) |
|---|---|---|---|---|---|---|---|
| 1 | 5 | 5 | 5 | 5 | 5 | 5 | 4 |
| 2 | 5 | 5 | 5 | 5 | 5 | 5 | 4 |

*) AVF Avena
*) ALM Alopecurus
*) SAL Setaria
*) LOM Lolium
*) ECG Echinochloa
*) AGR Agropyron repens
*) CND Cynodon dactylon

### Beispiel II : Nachauflaufbehandlung

Samen von Gräsern wurden in Töpfen ausgesät und im Gewächshaus angezogen. 3 Wochen nach der Aussaat wurden die als Spritzpulver bzw. als Emulsionskonzentrate formulierten erfindungsgemäßen Präparate in verschiedenen Dosierungen auf die Pflanzen gesprüht und nach 4 Wochen Standzeit im Gewächshaus die Wirkung der Präparate bonitiert.

Die erfindungsgemäßen Mittel waren gut gegen ein breites Spektrum von einjährigen Schadgräsern herbizid wirksam. Einige Präparate bekämpften ferner auch die mehrjährigen Schadgräser Cynodon dactylon, Sorghum halepense sowie Agropyron repens.

Tabelle II

Nachauflaufwirkung (Dosierung : 2,4 kg Aktivsubstanz/ha)

| Verbindung (Beispiel) | ALM | SAL | LOM | ECG |
|---|---|---|---|---|
| 1 | 5 | 5 | 5 | 5 |
| 2 | 5 | 5 | 5 | 5 |

Beispiel III : Kulturpflanzenverträglichkeit

In weiteren Versuchen im Gewächshaus wurden Samen einer größeren Anzahl von Kulturpflanzen in Töpfen ausgelegt. Ein Teil der Töpfe wurde sofort behandelt, die übrigen wurden im Gewächshaus aufgestellt, bis die Pflanzen 2 bis 3 echte Blätter entwickelt hatten und dann mit erfindungsgemäßen Substanzen besprüht.

Die Ergebnisse, die 4 bis 5 Wochen nach Applikation festgestellt wurden, zeigen, daß die erfindungsgemäßen Substanzen zweikeimblättrige Kulturen im Vor- und Nachauflauf-Verfahren selbst mit 2,4 kg/ha völlig oder fast völlig ungeschädigt lassen. Einige Substanzen schonen darüber hinaus auch Gramineen-Kulturen wie Gerste, Sorghum, Mais, Weizen oder Reis.

Die Substanzen sind somit bezüglich der in den vorigen Beispielen beschriebenen Unkrautwirkung hoch selektiv.

**Patentansprüche** (für die Vertragsstaaten : BE, CH, DE, FR, GB, IT, LI, NL)

1. Heterocyclisch substituierte 4-Phenoxyalkancarbonsäurederivate der allgemeinen Formel

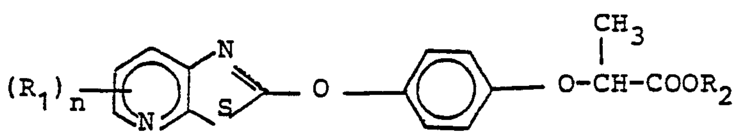

(I)

worin

$R_1$ Halogen,

n 0 bis 3,

$R_2$ H, $(C_1$-$C_{12})$-Alkyl,

oder ein Kationäquivalent einer organischen oder anorganischen Base bedeuten.

2. Verbindungen der Formel I, worin $R_1$ Wasserstoff oder Chlor in 5-Stellung und $R_2$ Wasserstoff oder $(C_1$-$C_4)$-Alkyl bedeutet.

3. Die Verbindung der Formel

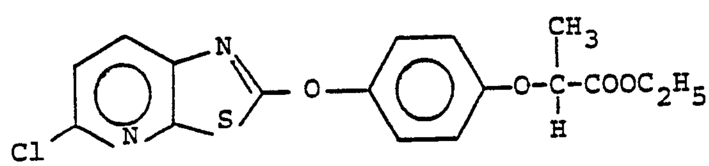

4. Die Verbindung der Formel

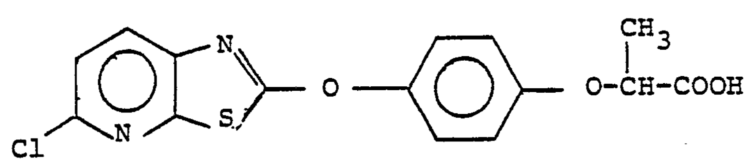

5. Die Verbindung der Formel

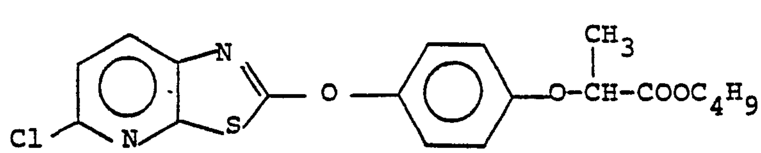

6. Verfahren zur Herstellung der Verbindungen der Formel I, dadurch gekennzeichnet, daß man

a) Verbindungen der Formel

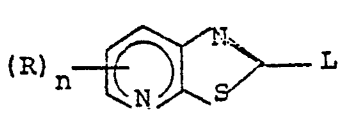

(II)

worin L eine Abgangsgruppe wie z. B. Halogen, $(C_1$-$C_4)$-Alkylsulfonyl, Phenylsulfonyl, $(C_1$-$C_4)$-Alkoxycarbonylmethylsulfonyl,$(C_1$-$C_4)$-Alkylsulfinyl, $(C_1$-$C_4)$-Alkylthio sowie den Mesyl- oder Tosylrest bedeutet, mit Verbindungen der Formel

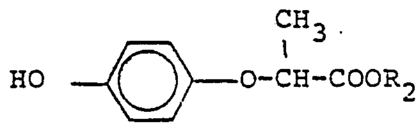

(III)

oder

b) Verbindungen der Formel

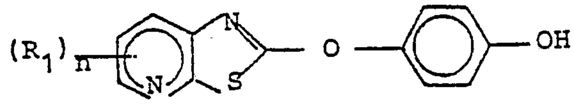

(IV)

mit Verbindungen der Formel

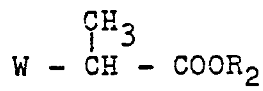

(V)

worin W für Halogen (vorzugsweise Chlor oder Brom) oder den Mesyl- bzw. Tosylrest steht, umsetzt oder

c) die nach Verfahren a) oder b) erhaltenen Verbindungen durch Verseifung, Salzbildung, Veresterung oder Umesterung in andere erfindungsgemäße Verbindungen der Formel I überführt.

7. Herbizide und wachstumsregulierende Mittel, gekennzeichnet durch einen Gehalt an einer Verbindung gemäß einem der Ansprüche 1 bis 5.

8. Verwendung von Verbindungen gemäß einem der Ansprüche 1 bis 5 zur Bekämpfung von Schadgräsern bzw. zur Wachstumsregulierung.

9. Verfahren zur Bekämpfung unerwünschten Pflanzenwuchses, dadurch gekennzeichnet, daß man eine wirksame Menge einer Verbindung gemäß einem der Ansprüche 1 bis 5 auf die Pflanzen bzw. die von ihnen bewachsene Fläche aufbringt.

10. Verfahren zur Wachstumsregulierung von Nutzpflanzen, dadurch gekennzeichnet, daß man auf die Nutzpflanzen eine wirksame Menge einer Verbindung gemäß einem der Ansprüche 1 bis 5 aufbringt.

**Patentansprüche** (für den Vertragsstaat AT)

1. Herbizide und wachstumsregulierende Mittel gekennzeichnet durch einen Gehalt an einer Verbindung der Formel I

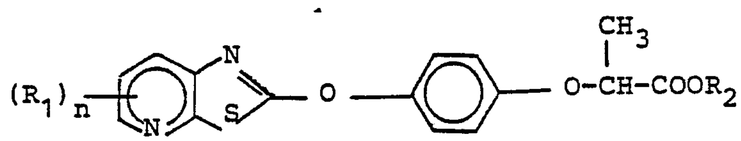

(I)

worin

$R_1$ Halogen,

n 0 bis 3,

$R_2$ H, $(C_1-C_{12})$-Alkyl,

oder ein Kationäquivalent einer organischen oder aorganischen Base bedeuten.

2. Verfahren zur Herstellung der Verbindungen der Formel I, dadurch gekennzeichnet, daß man

a) Verbindungen der Formel

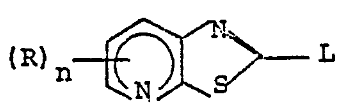

(II)

worin L eine Abgangsgruppe wie z. B. Halogen, $(C_1-C_4)$-Alkylsulfonyl, Phenylsulfonyl, $(C_1-C_4)$-Alkoxycarbonyl-methylsulfonyl, $(C_1-C_4)$-Alkylsulfinyl, $(C_1-C_4)$-Alkylthio sowie den Mesyl- oder Tosylrest bedeutet, mit Verbindungen der Formel

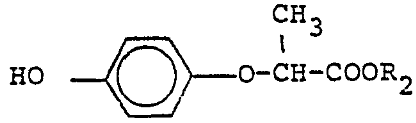

(III)

oder

b) Verbindungen der Formel

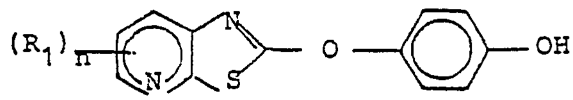

(IV)

mit Verbindungen der Formel

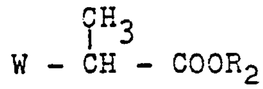

(V)

worin W für Halogen (vorzugsweise Chlor oder Brom) oder den Mesyl-bzw. Tosylrest steht, umsetzt oder

c) die nach Verfahren a) oder b) erhaltenen Verbindungen durch Verseifung, Salzbildung, Veresterung oder Umesterung in andere erfindungsgemäße Verbindungen der Formel I überführt.

3. Verwendung von Verbindungen gemäß Anspruch 1 zur Bekämpfung von Schadgräsern bzw. zur Wachstumsregulierung.

4. Verfahren zur Bekämpfung unerwünschten Pflanzenwuchses, dadurch gekennzeichnet, daß man eine wirksame Menge einer Verbindung gemäß Anspruch 1 auf die Pflanzen bzw. die von ihnen bewachsene Fläche aufbringt.

5. Verfahren zur Wachstumsregulierung von Nutzpflanzen, dadurch gekennzeichnet, daß man auf die Nutzpflanzen eine wirksame Menge einer Verbindung gemäß Anspruch 1 aufbringt.

**Claims** (for the Contracting States : BE, CH, DE, FR, GB, IT, LI, NL)

1. Heterocyclically substituted 4-phenoxyalkanecarboxylic acid derivatives having the formula

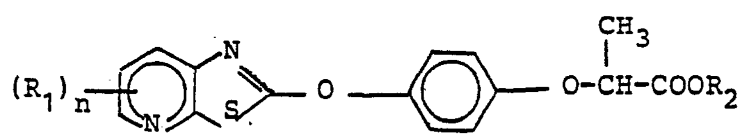

(I)

in which $R_1$ denotes halogen, n denotes 0 to 3, $R_2$ denotes H, ($C_1$-$C_{12}$)-alkyl or a cation equivalent of an organic or inorganic base.

2. A compound of the formula I in which R denotes hydrogen or chlorine in the 5-position and $R_2$ denotes hydrogen or ($C_1$-$C_4$)-alkyl.

3. The compound of the formula

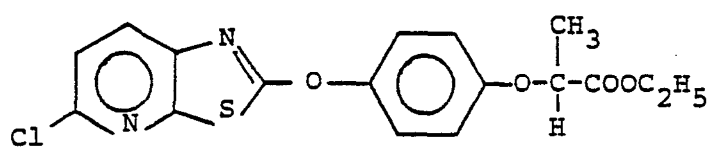

4. The compound of the formula

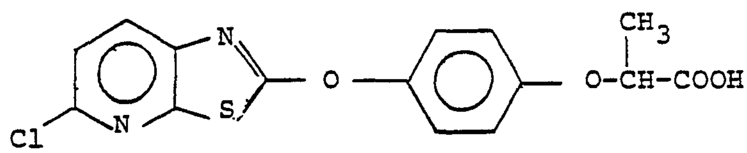

5. The compound of the formula

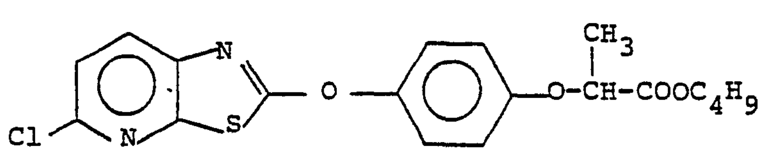

6. A process for the preparation of a compound of the formula I, which comprises

a) reacting compounds of the formula

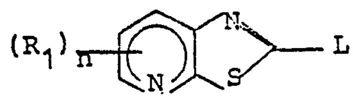

(II)

in which L is a detachable group, such as, for example, halogen, $(C_1-C_4)$-alkylsulfonyl, phenylsulfonyl, $(C_1-C_4)$-alkoxycarbonylmethylsulfonyl, $(C_1-C_4)$-alkylsulfinyl, $(C_1-C_4)$-alkylthio and the mesyl or tosyl radical, with compounds of the formula

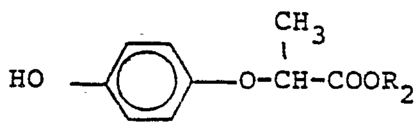

(III)

or

b) reacting compounds of the formula

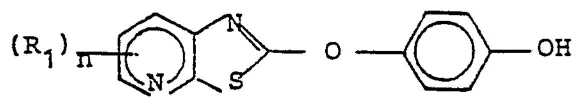

(IV)

with compounds of the formula

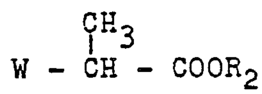

(V)

in which W represents halogen (preferably chlorine or bromine) or the mesyl or tosyl radical, or

c) converting the compounds obtained in accordance with processes a) or b) into other compounds, according to the invention, of the formula I by saponification, salt formation, esterification, or transesterification.

7. Herbicidal and growth-regulating agents with contain a compound as claimed in one of claims 1 to 5.

8. The use of compounds as claimed in one of claims 1 to 5 for combating graminaceous weeds or for regulating growth.

9. A process for combating undesirable plant growth, which comprises applying an effective quantity of a compound as claimed in one of claims 1 to 5 to the plants or to the area on which the latter grow.

10. A process for regulating the growth of useful plants, which comprises applying an effective quantity of a compound as claimed in one of claims 1 to 5 to the useful plants.

**Claims** (for the Contracting State AT)

1. Herbicidal and growth-regulating agents, comprising a compound of the formula

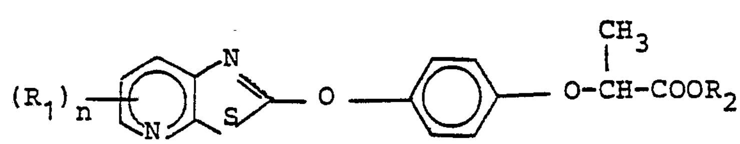

(I)

in which $R_1$ denotes halogen, n denotes 0 to 3, $R_2$ denotes H, $(C_1-C_{12})$-alkyl or a cation equivalent of an organic or inorganic base.

2. A process for the preparation of a compound of the formula I, which comprises

a) reacting compounds of the formula

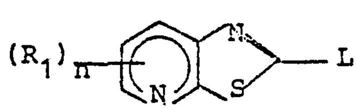

(II)

in which L is a detachable group, such as, for example, halogen $(C_1-C_4)$-alkylsulfonyl, phenylsulfonyl, $(C_1-C_4)$-alkoxycarbonylmethylsulfonyl, $(C_1-C_4)$-alkylsulfinyl, $(C_1-C_4)$-alkylthio and the mesyl or tosyl radical, with compounds of the formula

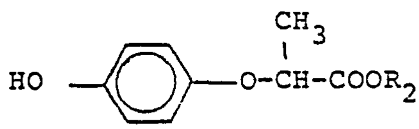

(III)

or

b) reacting compounds of the formula

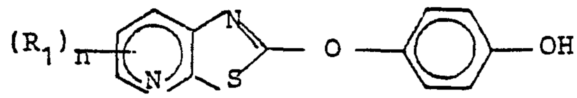

(IV)

with compounds of the formula

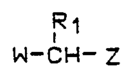

(V)

in which W represents halogen (preferably chlorine or bromine) or the mesyl or tosyl radical, or

c) converting the compounds obtained in accordance with processes a) or b) into other compounds, according to the invention, of the formula I by saponification, salt formation, esterification, or transesterification.

3. The use of compounds as claimed in claim 1 for combating graminaceous weeds or for regulating growth.

4. A process for combating undesirable plant growth, which comprises applying an effective quantity of a compound as claimed in claim 1 to the plants or to the area on which the latter grow.

5. A process for regulating the growth of useful plants, which comprises applying an effective quantity of a compound as claimed in claim 1 to the useful plants.

**Revendications** (pour les Etats contractants : BE, CH, DE, FR, GB, IT, LI, NL)

1. Dérivés d'acides phénoxyalcanecarboxyliques portant un substituant hétérocyclique sur la position 4 du noyau phényle, de formule générale

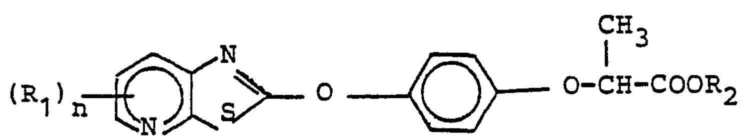

(I)

dans laquelle

$R_1$ est un halogène,

n vaut de 0 à 3,

$R_2$ est H ou un radical alkyle en $C_1$-$C_{12}$,

ou un équivalent cationique d'une base organique ou inorganique.

2. Composés de formule I, dans laquelle $R_1$ est l'hydrogène ou le chlore en position 5, et $R_2$ représente l'hydrogène ou (alkyle en $C_1$-$C_4$).

3. Le composé de formule

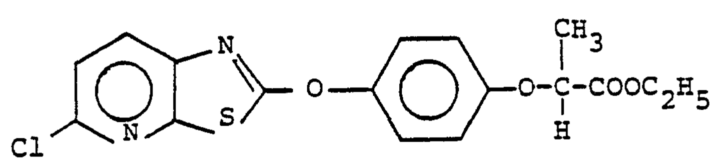

4. Le composé de formule

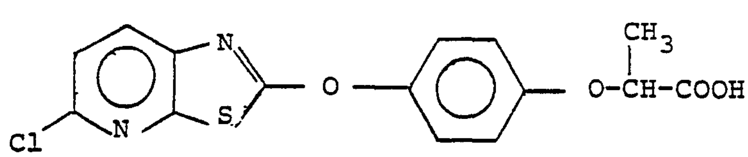

5. Le composé de formule

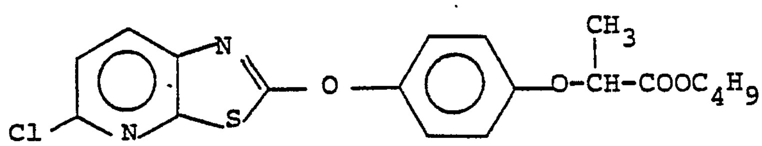

6. Procédé pour la préparation des composés de formule I, caractérisé en ce qu'on fait réagir

a) des composés de formule

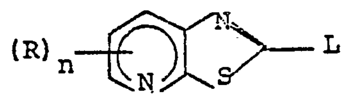

(II)

dans laquelle L est un groupe éliminable comme par exemple un halogène, un radical (alkyle en $C_1$-$C_4$)-sulfonyle, le radical phénylsulfonyle, un radical (alcoxy en $C_1$-$C_4$)-carbonyl-méthylsulfonyle, (alkyle en $C_1$-$C_4$)-sulfinyle ou (alkyle en $C_1$-$C_4$)-thio, ainsi que le radical mésyle ou tosyle, sur des composés de formule

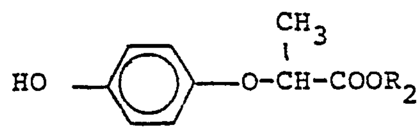

(III)

ou bien

b) des composés de formule

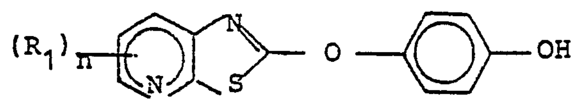

(IV)

sur des composés de formule

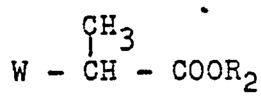

(V)

dans laquelle W est un halogène (de préférence le chlore ou le brome), ou le radical mésyle ou tosyle, ou bien

c) qu'on convertit en d'autres composés selon l'invention de formule I, par saponification, salification, estérification ou transestérification les composés obtenus par le procédé a) ou b).

7. Herbicides et substances de croissance, caractérisés en ce qu'ils contiennent un composé selon l'une des revendications 1 à 5.

8. Utilisation de composés selon l'une des revendications 1 à 5 pour la maîtrise des mauvaises herbes ou la régulation de leur croissance.

9. Procédé pour la maîtrise de la croissance de plantes indésirables, caractérisé en ce qu'on applique sur les plantes ou la surface sur laquelle elles poussent une quantité efficace d'un composé selon l'une des revendications 1 à 5.

10. Procédé pour la régulation de la croissance de plantes utiles, caractérisé en ce qu'on applique sur les plantes utiles une quantité efficace d'un composé selon l'une des revendications 1 à 5.

**Revendications** (pour l'Etat contractant AT)

1. Herbicides et substances de croissance, caractérisés en ce qu'ils contiennent un composé de formule I

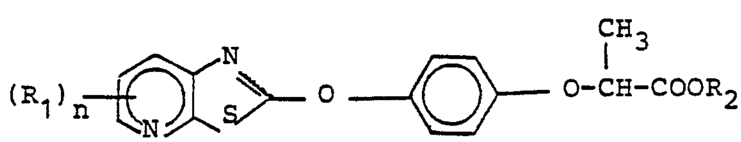

(I)

dans laquelle

$R_1$ est un halogène,

n vaut de 0 à 3,
$R_2$ est H ou un radical alkyle en $C_1$-$C_{12}$,
ou un équivalent cationique d'une base organique ou inorganique.

2. Procédé pour la préparation de composés de formule I, caractérisé en ce qu'on fait réagir

a) des composés de formule

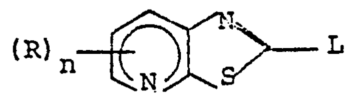

(II)

dans laquelle L est un groupe éliminable comme par exemple un halogène, un radical (alkyle en $C_1$-$C_4$)-sulfonyle, le radical phénylsulfonyle, un radical (alcoxy en $C_1$-$C_4$)-carbonylméthyl-sulfonyle, (alkyle en $C_1$-$C_4$)-sulfinyle, (alkyle en $C_1$-$C_4$)-thio, ainsi que le radical mésyle ou tosyle, sur des composés de formule

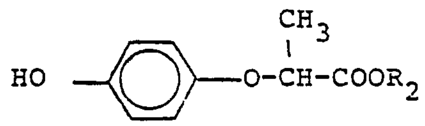

(III)

ou bien

b) des composés de formule

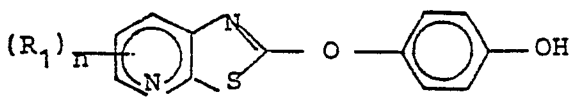

(IV)

sur des composés de formule

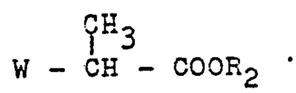

(V)

dans laquelle W est un halogène (de préférence le chlore ou le brome).ou le radical mésyle ou tosyle, ou bien

c) qu'on transforme en d'autres composés selon l'invention de formule I, par saponification, salification, estérification ou transestérification les composés obtenus selon les Exemples a) ou b).

3. Utilisation de composés selon la revendication 1 pour la maîtrise de mauvaises herbes ou pour la régulation de leur croissance.

4. Procédé pour la maîtrise de la croissance de plantes indésirables, caractérisé en ce qu'on applique sur les plantes ou sur la surface sur laquelle elles poussent une quantité efficace d'un composé selon la revendication 1.

5. Procédé pour la régulation de la croissance de plantes utiles, caractérisé en ce qu'on applique sur les plantes utiles une quantité efficace d'un composé selon la revendication 1.